# FASCICULE DE BREVET EUROPEEN

(11) **EP 2 063 796 B1**
(45) Date de publication et mention de la délivrance du brevet: **23.12.2009**
(21) Numéro de dépôt: 07823833.4
(22) Date de dépôt: 14.09.2007
(51) Int. Cl.: A61B 17/70

(54) **PROTHESE INTER-EPINEUSE LOMBAIRE ET SES APPLICATIONS**
LUMBARZWISCHENWIRBELPROTHESE UND IHRE ANWENDUNGEN
LUMBAR INTER-SPINE PROSTHESIS AND APPLICATIONS THEREOF

(30) Priorité: 18.09.2006 FR 0608155
(43) Date de publication de la demande: 03.06.2009
(73) Titulaire: Spineart SA, 1217 Meyrin (CH)
(72) Inventeur: MANGIONE, Paolo, F-33600 Pessac (FR); LEVIEUX, Jérôme, CH-1293 Bellevue (CH)
(74) Mandataire: Cabinet Plasseraud
(86) Numéro de dépôt international: PCT/FR2007/051938
(87) Numéro de publication internationale: WO 2008/034996

(56) Documents cités:
- EP-A- 0 743 045
- DE-A1- 4 000 086
- FR-A1- 2 722 980
- FR-A1- 2 774 581

## Description

La présente invention concerne une prothèse inter-épineuse lombaire et ses applications.

Au cours des deux dernières décennies, le traitement chirurgical de la pathologie dégénérative s'est largement répandu. A coté des gestes de simple décompression radiculaire (ablation de hernie, discectomie, recalibrage) et des gestes d'ostéosynthèse vertébrale, se sont développées plus récemment des techniques dites de « non fusion », soit sous forme de prothèses discales par voie antérieure, soit sous forme d'implants postérieurs assurant une certaine stabilisation mais autorisant la mobilité.

Certains de ces implants assurent cette stabilisation par un mécanisme de cale interépineuse, limitant surtout l'extension, associée ou non à un ligament qui entoure l'apophyse épineuse, et qui limite la flexion (des cales interépineuses sont commercialisés par la société Abbottspine sous la dénomination Wallis ® ou par la société Medtronic sous la dénomination Diam ®.

Bien que ces implants aient donné des résultats cliniques satisfaisants, ils ne restituent pas une cinétique normale au segment stabilisé.

On connaît déjà une cale inter-épineuse en titane en forme de U. (Voir brevet FR-A-2 722 980). Cette cale permet de stabiliser l'espace intervertébral et ainsi d'amoindrir le vieillissement du disque. Cependant ce système présente une raideur beaucoup trop importante pour restituer de façon harmonieuse les mouvement du rachis lombaire.

De plus, l'ancrage est unilatéral sur l'épineuse, il en résulte un fonctionnement moins efficace en flexion et une stabilité précaire dans l'espace.

Enfin, ce système impose au chirurgien un abord de chaque côté de l'épineuse, il est donc nécessaire de désinsérer des muscles dans une zone saine afin de pouvoir placer l'implant. En effet, la plupart du temps la compression radiculaire est uni-latérale. Le chirurgien n'aborde donc que d'un côté. Or, pour installer un dispositif de ce type, il sera obligé d'aborder les deux côtés, ce qui va augmenter la déstabilisation du segment rachidien. Par ailleurs il est très désagréable pour un chirurgien d'aborder une zone saine pour la seule raison d'installer un dispositif.

Il serait donc souhaitable de disposer d'un dispositif permettant de stabiliser le segment lombaire tout en restaurant une cinétique proche de la physiologie, c'est-à-dire comportant un centre de rotation situé un peu en dessous du plateau vertébral inférieur du segment instrumenté, à sa partie postérieure. De plus, il serait souhaitable qu'il puisse être installé en abordant le segment d'un seul côté de l'épineuse.

Après de longues recherches, la demanderesse a mis au point un nouveau type de prothèse inter-épineuse lombaire comportant un ressort de torsion (ressort de type « pince à linge » travaillant sur lui-même en rotation).

C'est pourquoi la présente demande a pour objet une prothèse inter-épineuse lombaire **caractérisée en ce qu**'elle comprend un ressort de torsion comportant deux branches, dont chacune des extrémités comporte un système de fixation à une apophyse d'une vertèbre.

Le ressort utilisé peut avoir des spires non jointives, mais les spires sont de préférence jointives.

Les branches du ressort de torsion sont prévues pour être fixées sur des apophyses de deux vertèbres adjacentes, en particulier des apophyses épineuses, notamment de vertèbres lombaires, l'axe du ressort étant décalé par rapport à la droite joignant les milieux des systèmes de fixation sur les apophyses.

Les extrémités des branches du ressort peuvent être accrochées directement aux apophyses ou notamment indirectement grâce à une pièce se fixant elle-même aux apophyses.

En vue d'une fixation directe, les extrémités des branches du ressort peuvent être simplement munies d'une boucle. Par exemple l'extrémité de chaque branche se replie en boucle pour former un crochet (ou pince). Dans une variante de réalisation, la boucle peut coopérer avec une vis installée dans l'apophyse.

En vue d'une fixation indirecte, les extrémités sont munies d'une pièce d'accrochage à l'apophyse sans perçage. Cette pièce d'accrochage est notamment une agrafe.

L'agrafe comprend par exemple une lame allongée généralement plate dont les extrémités sont recourbées du même côté pour former une pince pour s'accrocher sur l'apophyse à laquelle elle est destinée. L'espacement entre les parties recourbées est adapté à la taille de l'apophyse. De préférence la lame allongée a une forme en Y pour constituer deux crochets d'un côté et un seul de l'autre.

La lame peut être constituée de plusieurs pièces. Notamment elle comprend deux parties coulissantes, une située du côté des spires du ressort et nommée "interne" et une située du côté opposé aux spires du ressort et nommée "externe" Ainsi, pour l'installation, on peut allonger par coulissement par exemple la lame externe, puis rapprocher les crochets de chacune des deux parties coulissantes pour venir pincer l'apophyse. On peut alors bloquer la pince ainsi formée. Pour ce faire, par exemple la partie coulissante externe peut comprendre une lumière longitudinale allongée tandis que la partie coulissante interne peut comprendre un boulon fileté pour guider et bloquer la pince sur l'apophyse à l'aide d'un écrou ou d'un filetage ménagé dans la partie coulissante interne, ou inversement. Une branche peut aussi constituer une glissière pour l'autre branche. Pour cela, ses bords longitudinaux peuvent être recourbés pour former un canal ouvert pour l'autre branche.

La coopération entre le ressort et le dispositif d'accrochage à l'apophyse sans perçage peut être réalisé par tout moyen connu, de préférence autorisant la rotation relative des deux pièces. Dans des conditions préférentielles de réalisation, la face de la lame allongée opposée à celle formant un crochet, comprend un téton permettant la fixation de l'extrémité d'une branche du ressort, par exemple grâce à une boucle prévue à ce niveau, laissant libre la rotation. Le téton est avantageusement muni d'un épaulement pour prendre en sandwich une branche du ressort entre ledit épaulement et la lame allongée. A l'inverse, le téton peut être muni d'une gorge dans laquelle vient s'installer une boucle prévue à l'extrémité d'une branche du ressort.

Dans d'autres conditions préférentielles de mise en oeuvre de l'invention, une des branches du ressort est pliée une première fois parallèlement à l'axe du ressort, à la sortie des spires, et une seconde fois à l'autre bout des spires, à angle droit, de sorte que les deux branches sont dans le même plan et que l'axe du ressort est perpendiculaire à ce plan.

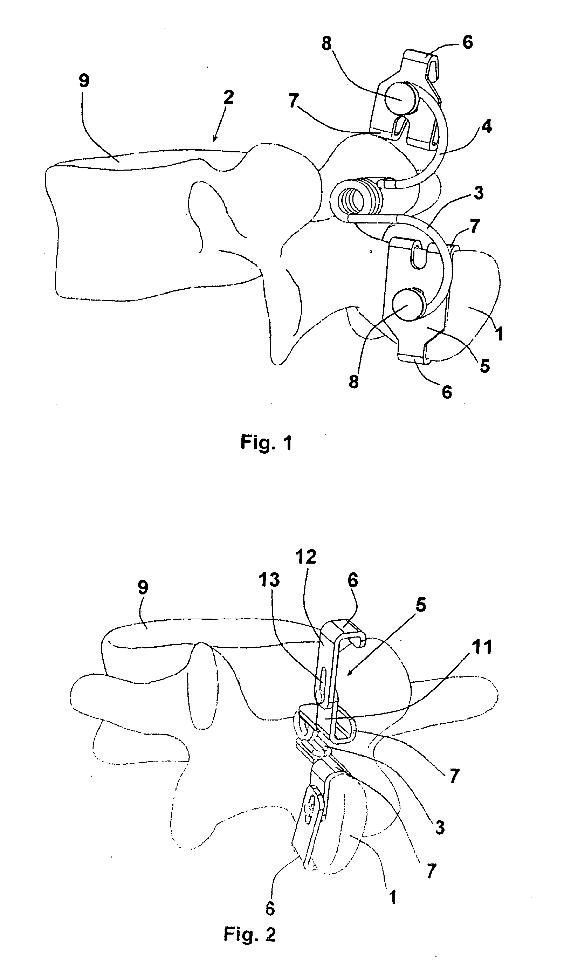

Dans encore d'autres conditions préférentielles de mise en oeuvre de l'invention, les deux branches sont courbées sur plus de 50 % de leur longueur comme illustré sur la figure 1 ou alors sont recourbées en épingle à cheveux comme illustré sur la figure 2.

En général, le ressort sera réalisé en fil rond. Il sera aussi notamment réalisé en matériau métallique convenable pour une implantation. Il est de préférence réalisé en Titane ou alliage de Titane. Les éventuels autres éléments de la prothèse inter-épineuse sont avantageusement réalisés dans les mêmes matériaux.

Le diamètre extérieur du ressort est avantageusement de 3 à 13, de préférence de 4 à 12, notamment de 5 à 11, tout particulièrement de 6 à 10 mm.

Le diamètre du fil constitutif du ressort est avantageusement de 1,0 à 4,5, de préférence de 1,0 à 4,0, notamment de 1,5 à 3,5, tout particulièrement de 2 à 3 mm.

L'axe du ressort est décalé par rapport à la droite joignant les milieux des crochets externes d'ancrage sur les apophyses de préférence de 3 à 18, notamment de 4 à 16, tout particulièrement de 5 à 14 mm au repos.

Les prothèses inter-épineuses objets de la présente invention possèdent de très intéressantes propriétés et qualités. Elles sont notamment capables de restituer au segment de la colonne vertébrale instrumenté une cinétique proche de la physiologie, c'est-à-dire un centre de rotation situé un peu en dessous du plateau vertébral de la vertèbre inférieure du segment instrumenté, et à sa partie postérieure.

Lorsque le ressort est compressé, les crochets peuvent êtres placés entre les épineuses par un abord unilatéral. Le geste chirurgical est donc facilité. De plus cela évite au chirurgien d'aborder la colonne des deux côtés de l'épineuse afin de limiter la déstabilisation du segment et de laisser intacte une zone saine.

Le mouvement du ressort possède un centre instantané de rotation proche de celui d'une personne saine (sous le plateau de la vertèbre inférieure, dans le tiers postérieur).

La souplesse du type de ressort utilisé permet un mouvement aisé et harmonieux.

Les agrafes à crochets optionnelles permettent un ancrage fiable ainsi qu'un fonctionnement en extension et en flexion.

Ces propriétés sont illustrées ci-après dans la partie expérimentale. Elles justifient l'utilisation des prothèses inter-épineuses ci-dessus décrites, dans le traitement de pathologies lombaires dégénératives

C'est pourquoi la présente demande a aussi pour objet un procédé de stabilisation d'une articulation lombaire **caractérisé en ce que** l'on installe entre deux vertèbres, de préférence lombaires, une prothèse inter-épineuse ci-dessus.

Notamment on rapproche les branches du ressort à l'aide d'un instrument ancillaire approprié, on fixe les extrémités des branches sur les apophyses notamment épineuses de deux vertèbres adjacentes et on libère les branches.

Les conditions préférentielles de mise en oeuvre des prothèses inter-épineuses ci-dessus décrites s'appliquent également aux autres objets de l'invention visés ci-dessus, notamment aux procédés de stabilisation d'une articulation lombaire.

L'invention sera mieux comprise si l'on se réfère aux dessins annexés sur lesquels
- la figure 1 représente une vue latérale d'une prothèse inter-épineuse munie d'agrafes et montée sur une vertèbre lombaire
- la figure 2 représente une vue frontale en élévation d'une autre prothèse inter-épineuse munie d'agrafes et montée sur une vertèbre lombaire
- la figure 3 représente une vue latérale en élévation d'une prothèse inter-épineuse munie d'agrafes et montée sur deux vertèbres lombaires, schématisant l'axe de rotation de la prothèse.
- la figure 4 représente une vue latérale en élévation d'une variante de prothèse inter-épineuse dont les extrémités des branches du ressort sont directement accrochées aux apophyses.

Sur la figure 1, on observe une prothèse inter-épineuse selon l'invention munie d'agrafes et montée sur l'apophyse épineuse 1 d'une vertèbre lombaire 2.

La prothèse inter-épineuse représentée comprend un ressort de torsion comportant deux branches 3 et 4. Le ressort utilisé a des spires jointives. Chacune des extrémités des deux branches 3 et 4 comporte un système de fixation à une apophyse d'une vertèbre qui est une agrafe 5. L'agrafe 5 comprend une lame allongée généralement plate dont les extrémités sont recourbées du même côté pour former un crochet 6,7 pour s'accrocher sur l'apophyse 1. L'espacement entre les parties recourbées 6,7 est adapté à la taille de l'apophyse. La lame allongée a une forme en Y pour constituer deux crochets 7 d'un côté et un seul crochet 6 de l'autre.

La branche supérieure 4 du ressort est pliée une première fois parallèlement à l'axe du ressort, vers la gauche, à la sortie des spires, et une seconde fois à l'autre bout des spires, du côté droit et à angle droit, de sorte que les deux branches 3,4 sont essentiellement situées dans un même plan et que l'axe du ressort est perpendiculaire à ce plan.

La face de la lame allongée opposée à celle formant crochet, comprend un téton 8 permettant la fixation de l'extrémité d'une branche 3,4 du ressort, grâce à une boucle prévue à ce niveau. Celle-ci laisse libre la rotation relative entre les deux pièces.

On peut observer que l'axe du ressort est espacé de la droite joignant le centre des crochets 6 (décalé par rapport à celle-ci).

Les deux branches 3,4 sont courbées en arc de cercle, presque à partir des spires du ressort, sur plus de 50 % de leur longueur.

Les branches et les agrafes sont agencées de telle sorte que le ressort se trouve placé sous le plateau vertébral 9. Ainsi, la prothèse inter-épineuse de l'invention possède un centre de rotation situé un peu en dessous du plateau vertébral 9 de la vertèbre inférieure 2 du segment instrumenté, et à sa partie postérieure (la vertèbre supérieure sur laquelle s'attache la seconde agrafe n'est pas représentée).

Les tétons 8 sont munis d'un épaulement vers l'avant de la figure pour prendre en sandwich les branches 3,4 du ressort entre ledit épaulement et la lame allongée de l'agrafe 5. La lame allongée, du côté opposé à celui comprenant le téton 8, est repliée vers la droite pour former des crochets 6,7, un crochet 6 situé vers le haut et deux crochets 7 situés vers le bas pour l'agrafe supérieure et l'inverse pour l'agrafe inférieure.

La figure 2 représente une variante de l'invention. Pour éviter d'avoir à replier les crochets sur les apophyses, l'agrafe 5 comprend aussi une lame allongée généralement plate dont les extrémités sont recourbées du même côté pour former un crochet 6,7 pour s'accrocher sur l'apophyse 1, mais la lame est constituée de plusieurs pièces.

Elle comprend deux parties coulissantes, interne 11, et externe 12. Ainsi, pour l'installation on peut allonger l'agrafe 5 par coulissement de la partie de lame externe, puis rapprocher les crochets de chacune des deux parties coulissantes pour venir pincer l'apophyse. On peut alors bloquer la pince ainsi formée. La partie coulissante externe 12 comprend une lumière longitudinale allongée 13 tandis que la partie interne 11 comprend un boulon fileté pour guider et bloquer la pince sur l'apophyse 1 par coopération avec un filetage prévu dans la partie interne 11 de la lame. Le vissage du boulon fileté dans la partie interne 11 de la lame bloque le coulissement et rend l'agrafe 5 solidaire de l'apophyse 1.

Dans cette variante, les deux branches 3 et 4 du ressort sont soudées à la partie interne 11 de l'agrafe 5.

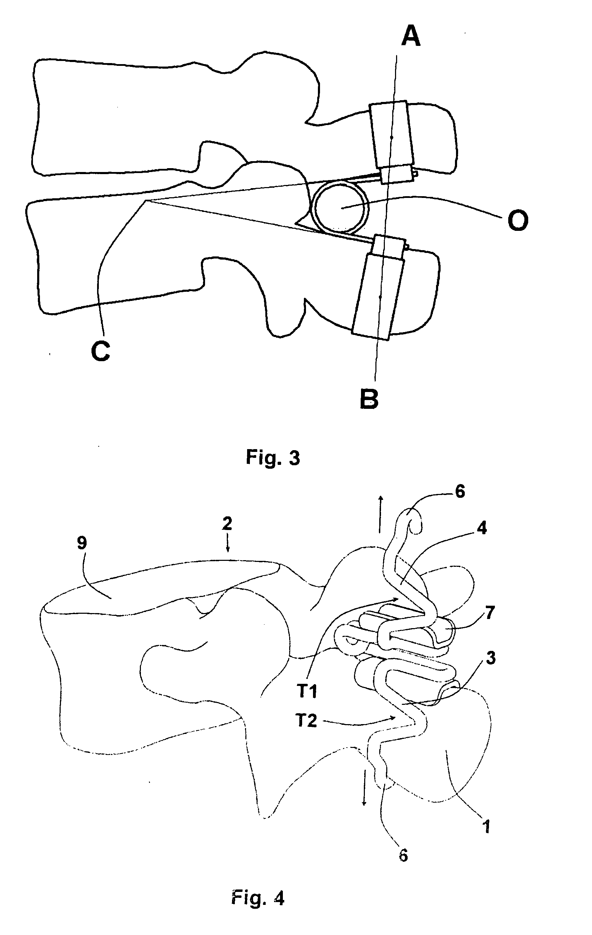

Comme on le voit sur la figure 3, le ressort possède un centre instantané de rotation C proche de celui d'une personne saine (sous le plateau de la vertèbre inférieure, dans le tiers postérieur). En outre, l'axe O du ressort est décalé par rapport à la droite AB joignant les centres des crochets de fixation externes de la prothèse sur les apophyses.

Sur la figure 4, on observe sensiblement les mêmes éléments que sur les figures 1 et 2. Dans la variante réalisée, à la sortie du ressort, les branches 3 et 4 sont repliées en épingle à cheveux et comportent une pièce en forme de cuvette allongée servant de premier crochet 7, interne. Dans cette variante, cette pièce a été soudée sur les branches au niveau de l'épingle. Les branches se poursuivent vers l'extérieur en formant dans un premier temps un V (formant ainsi 2 triangles ouverts T1 et T2) dont l'extrémité externe de la branche se replie en boucle, parallèlement à l'axe du ressort, pour former le second crochet 6, externe. Les parties en V des branches sont sensiblement dans un même plan, ledit plan étant perpendiculaire à l'axe du ressort.

Dans cette variante, les branches 3 et 4, parce qu'elles sont pliées en V, ont une longueur qui leur confère une élasticité suffisante pour permettre d'écarter l'un de l'autre les crochets 6 et 7 (comme représenté par les deux flèches verticales). Une pince peut être utilisée à cette fin. Le relâchement de la pince permet la fixation sûre de la prothèse sur les apophyses 1. Il va de soi qu'une forme courbe des branches 3 et 4, comme celle représentée à la figure 1 convient également.

## Revendications

1. Prothèse inter-épineuse lombaire **caractérisée en ce qu'**elle comprend un ressort de torsion comportant deux branches (3,4), dont chacune des extrémités comporte un système de fixation (5) à une apophyse (1) d'une vertèbre (2).

2. Prothèse inter-épineuse selon la revendication 1, **caractérisée en ce que** l'axe (O) du ressort est décalé par rapport à la droite joignant les milieux des systèmes de fixation sur les apophyses.

3. Prothèse inter-épineuse selon la revendication 1 ou 2, **caractérisée en ce que** les extrémités des branches (3,4) du ressort sont accrochées aux apophyses (1) indirectement grâce à une pièce d'accrochage (5) se fixant elle-même aux apophyses (1).

4. Prothèse inter-épineuse selon la revendication 3, **caractérisée en ce que** la pièce d'accrochage (5) est une agrafe comprenant une lame allongée généralement plate dont les extrémités sont recourbées du même côté pour former un crochet (6,7) pour s'accrocher sur l'apophyse (1) à laquelle elle est destinée.

5. Prothèse inter-épineuse selon la revendication 4, **caractérisée en ce que** la lame allongée a une forme en Y pour constituer deux crochets (7) d'un côté et un seul (6) de l'autre.

6. Prothèse inter-épineuse selon l'une des revendications 4 et 5, **caractérisée en ce que** la lame comprend deux parties coulissantes.

7. Prothèse inter- épineuse selon la revendication 1 ou 2, **caractérisée en ce que** les extrémités des branches (3,4) du ressort sont directement accrochées aux apophyses (1)

8. Prothèse inter-épineuse selon la revendication 1 ou 2, **caractérisée en ce que** les extrémités des branches (3,4) du ressort sont directement accrochées aux apophyses (1) et **en ce que** l'extrémité de chaque branche (3,4) se replie en boucle pour former un crochet (6).

9. Prothèse inter-épineuse selon l'une des revendications 1 à 8, **caractérisée en ce que** le diamètre extérieur du ressort est de 3 à 13 mm.

10. Prothèse inter-épineuse selon l'une des revendications 1 à 9, **caractérisée en ce que** le diamètre du fil constitutif du ressort est de 1 à 4 mm.

11. prothèse inter-épineuse selon l'une des revendications 1 à 10, **caractérisée en ce que** l'axe (O) du ressort est décalé par rapport à la droite joignant les milieux des systèmes de fixation sur les apophyses (1) de 4 à 16 mm au repos.

## Claims

1. A lumbar interspinous prosthesis, **characterized in that** it comprises a torsion spring with two branches (3, 4), each end of which comprises a system (5) for fixation to an apophysis (1) of a vertebra (2).

2. The interspinous prosthesis according to claim 1, **characterized in that** the axis (O) of the spring is offset with respect to the straight line joining the centres of the systems for fixation to the apophyses.

3. The interspinous prosthesis according to claim 1 or 2, **characterized in that** the ends of the branches (3, 4) of the spring are fastened to the apophyses (1) indirectly by way of a fastening component (5) which fixes itself to the apophyses (1).

4. The interspinous prosthesis according to claim 3, **characterized in that** the fastening component (5) is a clip comprising a generally flat elongate blade whose ends are curved on the same side in order to form a hook (6, 7) for fastening to the apophysis (1) for which it is intended.

5. The interspinous prosthesis according to claim 4, **characterized in that** the elongate blade has a Y shape in order to form two hooks (7) at one end and a single hook (6) at the other end.

6. The interspinous prosthesis according to one of claims 4 and 5, **characterized in that** the blade comprises two sliding parts.

7. The interspinous prosthesis according to claim 1 or 2, **characterized in that** the ends of the branches (3, 4) of the spring are fastened directly to the apophyses (1).

8. The interspinous prosthesis according to claim 1 or 2, **characterized in that** the ends of the branches (3, 4) of the spring are fastened directly to the apophyses (1) and **in that** the end of each branch (3, 4) folds back in a loop shape in order to form a hook (6).

9. The interspinous prosthesis according to one of claims 1 to 8, **characterized in that** the external diameter of the spring Is 3 to 13 mm.

10. The interspinous prosthesis according to one of claims 1 to 9, **characterized in that** the diameter of the wire from which the spring is made Is 1 to 4 mm.

11. The interspinous prosthesis according to one of claims 1 to 10, **characterized in that** the axis (O) of the spring, at rest, is offset with respect to the straight line joining the centres of the systems for fixation to the apophyses (1) by 4 to 16 mm.

## Patentansprüche

1. Lumbarzwischenwlrbelprothese **dadurch gekennzeichnet, dass** sie eine Torsionsfeder umfasst, die zwei Zweige (3,4) beinhaltet, deren Jeweiliges Ende ein Befestigungssystem (5) an einer Apophyse (1) eines Wirbels (2) beinhaltet.

2. Lumbarzwischenwirbeiprothese nach Anspruch 1, **dadurch gekennzeichnet, dass** die Achse (O) der Feder versetzt Ist hinsichtlich der Geraden, die die Mitten der Befestigungssysteme auf den Apophysen verbindet.

3. Lumbarzwischenwirbelprothese nach Anspruch 1 oder 2, **dadurch gekennzeichnet, dass** die Enden der Zweige (3,4) der Feder indirekt an den Apophysen (1) befestigt sind mittels eines Befestigungsstückes (5), das sich selbst an den Apophysen (1) fixiert.

4. Lumbarzwischenwirbelprothese nach Anspruch 3, **dadurch gekennzeichnet, dass** das Befestigungsstück (5) eine Klammer ist, die eine im Allgemeinen flache langgestreckte Platte umfasst, deren Enden von derselben Seite aus umgebogen werden, um einen Haken (6,7) zu bilden, um sich auf der Apophyse (1) zu befestigen, für die sie bestimmt Ist.

5. Lumbarzwischenwirbelprothese nach Anspruch 4, **dadurch gekennzeichnet, dass** die langgestreckte Platte eine Y-Form hat, um zwei Haken (7) auf einer Seite und einen einzigen Haken (6) auf der anderen zu bilden.

6. Lumbarzwischenwlrbelprothese nach einem der Ansprüche 4 und 5, **dadurch gekennzeichnet, dass** die Platte zwei Schiebetelle umfasst.

7. Lumbarzwischenwirbeiprothese nach einem der Ansprüche 1 oder 2, **dadurch gekennzeichnet, dass** die Enden der Zweige (3,4) der Feder direkt an den Apophysen (1) befestigt werden.

8. Lumbarzwischenwirbeiprothese nach einem der Ansprüche 1 oder 2, **dadurch gekennzeichnet, dass** die Enden der Zweige (3,4) der Feder direkt an den Apophysen (1) befestigt werden und **dadurch** dass das Ende jedes Zweiges (3,4) sich zu einer Schleife biegt, um einen Haken (6) zu bilden.

9. Lumbarzwischenwirbeiprothese nach einem der Ansprüche 1 bis 8, **dadurch gekennzeichnet, dass** der Außendurchmesser der Feder von 3 bis 13 mm beiträgt.

10. Lumbarzwischenwirbeiprothese nach einem der Ansprüche 1 bis 9, **dadurch gekennzeichnet, dass** der Durchmesser des konstitutiven Drahtes der Feder von 1 bis 4 mm beträgt.

11. Lumbarzwischenwirbeiprothese nach einem der Ansprüche 1 bis 10, **dadurch gekennzeichnet, dass** die Achse (0) der Feder von 4 bis 18 mm Im Ruhezustand versetzt Ist hinsichtlich der Geraden, die die Mitten der Befestigungssysteme auf den Apophysen (1) verbindet.
